Europäisches Patentamt

European Patent Office

Office européen des brevets

(19)

(11) Publication number: **0 521 016 B1**

# EUROPEAN PATENT SPECIFICATION

(45) Date of publication of patent specification: **28.06.95** (51) Int. Cl.6: **A61F 13/15**

(21) Application number: **91905693.7**

(22) Date of filing: **20.03.91**

(86) International application number:
**PCT/US91/01780**

(87) International publication number:
**WO 91/14414 (03.10.91 91/23)**

(54) **NONWOVEN FABRIC FOR DIAPER TOP SHEET AND METHOD OF MAKING SAME.**

(30) Priority: **21.03.90 US 496600**

(43) Date of publication of application:
**07.01.93 Bulletin 93/01**

(45) Publication of the grant of the patent:
**28.06.95 Bulletin 95/26**

(84) Designated Contracting States:
**DE FR GB IT SE**

(56) References cited:
**EP-A- 0 235 309**
**WO-A-86/01400**
**FR-A- 2 588 285**
**GB-A- 2 055 690**
**US-A- 4 077 410**

(73) Proprietor: **FIBERWEB NORTH AMERICA, INC.**
**840 Southeast Main Street**
**Simpsonville, SC 29681 (US)**

(72) Inventor: **NEWKIRK, David, D.**
**844 SE Main Street**
**Simpsonville, SC 29861 (US)**

(74) Representative: **Patentanwälte Grünecker,**
**Kinkeldey, Stockmair & Partner**
**Maximilianstrasse 58**
**D-80538 München (DE)**

## Description

Technical Field

The present invention relates to disposable absorbent pads useful as disposable diapers, adult incontinence pads, sanitary napkins and the like. More particularly, the present invention relates to a nonwoven fabric top sheet for use in such absorbent pads.

Background Art

Absorbent pads useful as disposable diapers customarily comprise a water-resistant backing sheet, a layer of absorbent or superabsorbent material, and a liquid pervious top sheet (often referred to as a coverstock, or, in diaper applications, a diaper liner) which is placed in contact with the body of the wearer.

To be useful as a top sheet in such a construction, the sheet material should provide fast initial passage of the liquid to be absorbed through the top sheet into the layer of absorbent material (i.e., exhibit a short "strike-through" time), while at the same time delaying or minimizing passage of the absorbed liquids from the layer of absorbent material back through the top sheet to the skin of the wearer (i.e., exhibit high "dryness" or low "surface rewet"). In addition, it is highly desirable that the top sheet material be flexible and soft to the touch. Furthermore, the top sheet material must have sufficient strength not to tear or rupture when wet.

Many different top sheet constructions have been disclosed for the manufacture of such absorbent articles, each typically being claimed to provide an improvement in one or more of the characteristics noted above. In the past, the top sheets typically comprised one or more nonwoven webs of natural or synthetic textile fibers such as rayon, polyamide, polyester, polypropylene, or the like, which were stabilized and secured together by a cured binder composition included within the webs. For example, US-A-4,332,253 (Schoots) discloses such a top sheet wherein the nonwoven webs are stabilized and secured together with a polymeric binder composition containing at least 1% by weight of combined 2-ethyl hexyl acrylate. The inclusion of the 2-ethyl hexyl acrylate reportedly improves the dryness of the top sheet.

Similarly, US-A-4,377,615 (Suzuki et al.) discloses a two-layer top sheet in which the nonwoven webs are stabilized and adhered together by an adhesive binder composition. The upper layer of the top sheet containing, as a principal element thereof, hydrophobic fibers and the lower layer containing a mixture of hydrophobic fibers and hydrophilic fibers, with the fibers in the Lower layer being of coarser denier than the fibers in the upper layer, and the lower layer containing a smaller amount of the adhesive bonding material than the upper layer. This construction reportedly provides a top sheet exhibiting superior strike-through and dryness properties, an upper layer which is excellent in smoothness, touch and strength, and a lower layer which is excellent in bulkiness and cushion.

Currently, however, most top sheets are thin, low basis weight, carded or spunbond nonwoven fabrics formed by thermally bonding together synthetic thermoplastic fibers that have been made somewhat hydrophilic by the addition of wetting agents. In this regard, US-A-4,668,566 (Braun) discloses a two-layer top sheet comprising a first layer of polypropylene fibers thermally bonded to a second layer of polyethylene fibers, at discrete locations, which is formed by passing the layers between heated rolls, one having a smooth surface and one having a raised pattern thereon. Reportedly, a top sheet made in this manner exhibits increased softness and tensile strength. However, in order to obtain good strike-through, the polyethylene filaments must be coated with a wetting agent to increase their hydrophilicity.

Similarly, US-A-4,704,112/EP-A-0,235,309 (Suzuki et al.) discloses a two-layer top sheet comprising a first layer, having a pattern of apertures, composed of hydrophobic fibers in an amount of 70 to 100% by weight and hydrophilic fibers in an amount of 0 to 30% by weight, thermally bonded to a second layer, having no apertures, composed of hydrophilic fibers in an amount of 50 to 100% by weight and hydrophobic fibers in an amount of 0 to 50% by weight. Useful hydrophobic fibers are said to include polyester, polypropylene, polyethylene, acryl and polyurethane fibers, and useful hydrophilic fibers are said to include rayon fibers, cotton fibers and synthetic fibers such as polyester in which the fiber surface has been imparted with a hydrophilic nature. Reportedly, such a top sheet construction provides an improvement in the balance achieved between surface rewet and strike-through characteristics.

Although the top sheet constructions referred to above indicate that significant advances have been made in the formulation of top sheets exhibiting the desired characteristics, there remain significant disadvantages associated with each of these constructions. For example, the adhesive binder compositions included within the adhesively bonded constructions increase the cost of the top sheet. Additionally, since these binder resin compositions are often applied in the form of aqueous dispersions, the energy required for drying the nonwoven webs and curing the binder resin further increases the cost of manufacture. Moreover, while the thermally bonded constructions avoid the disadvantages associated with the use of adhesive binder resins, they undesirably require the use of hydrophobic thermoplastic fibers which introduce at least three disadvantages. First, the hydrophobic thermoplastic fibers must be treated with wetting agents to provide sufficient hydrophilicity to achieve adequate strike-through. Second synthetic thermoplastic fibers are often judged to provide less comfort than natural hydrophilic fibers. Third, the hydrophobic thermoplastic fibers are not biodegradable, and, thus, contribute to the growing environmental problems of waste disposal and management.

It is, therefore, an object of the present invention to provide a thermally bonded nonwoven top sheet that provides a superior balance of strike-through and dryness properties, and which comprises a significant proportion of natural, biodegradable, hydrophilic fibers.

Summary of the Invention

The present invention provides a two-layer, nonwoven fabric ideally suited for use as a top sheet in absorbent articles such as disposable diapers and the like. The fabric comprises a continuous first layer, comprising at least about 75 weight percent hydrophobic thermoplastic fibers, and a second layer, comprising a blend of from about 20 to about 70 weight percent hydrophobic thermoplastic fibers and from about 30 to about 80 weight percent natural hydrophilic fibers. The layers are secured together by bonds formed of melt-fused portions of the hydrophobic thermoplastic fibers.

The unique two-layer construction of the present invention provides a flexible, comfortable fabric having a smooth soft surface for application against the body, a superior balance of strike-through and surface rewet properties, and sufficient tensile strength to be puncture and tear resistant even when wet. Furthermore, all of these desirable attributes of the fabric have been obtained with the inclusion of a significant proportion of natural, biodegradable, hydrophilic fibers, and without the inclusion of binder resins to stabilize and secure the layers together.

Moreover, with respect to the balance of strike-through and surface rewet properties, Applicant unexpectedly discovered that the two-layer construction of the invention exhibits a superior balance of strike-through and surface rewet properties in comparison to a single-layer, thermally bonded fabric having about the same overall ratio of hydrophobic fibers to natural hydrophilic fibers, but composed of a homogeneous blend of the hydrophobic and natural hydrophilic fibers. While the reasons for the improvement in both of these properties is not precisely known, it is apparent that the superior balance of these properties exhibited by the multi-layer construction results from the difference in the compositions of the respective layers. Accordingly, it is believed that the first layer must be composed of at least about 75 weight percent hydrophobic thermoplastic fibers, and the second layer must be composed of a blend of hydrophobic thermoplastic fibers and natural hydrophilic fibers wherein the natural hydrophilic fibers account for at least about 30 weight percent of the layer, in order to provide the resulting fabric with this unexpectedly superior balance of short strike-through times and low surface rewet characteristics. Furthermore, the fibrous blend of the second layer must be composed of at least about 20 weight percent hydrophobic thermoplastic fibers in order to provide the resulting thermally bonded fabric with sufficient strength to be useful as a top sheet.

To obtain a top sheet of the invention exhibiting an even better balance of strength, strike-through and surface rewet properties, it is generally preferred that the first layer be composed of at least about 95% by weight hydrophobic thermoplastic fibers, and the second layer be composed of a blend having a weight ratio of thermoplastic hydrophobic fibers to natural hydrophilic fibers in the range of from about 25:75 to about 50:50. In this regard, it is even more preferred for the first layer to be about 100 percent hydrophobic thermoplastic fibers.

Description of the Preferred Embodiments

The two-layer, nonwoven fabric of the invention may be formed in a relatively simple and economical manner by a method comprising the steps of

(a) forming a first, continuous, nonwoven, fibrous web comprising at least about 75 weight percent hydrophobic thermoplastic fibers;

(b) forming a second, nonwoven, fibrous web comprising a blend of from about 20 to about 70 weight percent hydrophobic thermoplastic fibers and from about 30 to about 80 weight percent natural hydrophilic fibers;

3

(c) forming a composite web by arranging the first and second webs in an overlying adjacent relationship;

(d) thermally bonding the first and second nonwoven webs together by causing melt-fusing of the hydrophobic thermoplastic fibers; and

(e) allowing the composite web to cool to resolidify the melt-fused thermoplastic fibers and strengthen the bonds formed between the layers.

The nonwoven webs formed in steps (a) and (b) of the above recited method may be prepared from staple fibers by conventional means such as air laying and carding techniques. Alternatively, when the web formed in step (a) consists entirely of hydrophobic thermoplastic fibers, it can be formed from continuous hydrophobic thermoplastic fibers by the spunbond process. Webs formed from continuous thermoplastic fibers by the spunbond process are well known in the art as disclosed in US-A-3,692,618; US-A-4,041,203; US-A-4,405,297 and US-A-4,753,834, the disclosures of which are incorporated herein by reference.

The fibers used in the nonwoven webs formed in steps (a) and (b) are preferably of a size ranging from about 1.1 to 6.6 dtex [1 to 6 denier], more preferably from about 1.1 to 3.3 dtex [1 to 3 denier], with the most preferred fibers ranging in size from about 1.7 to 2.8 dtex [1.5 to 2.5 denier]. Typically, the smaller the fiber the softer the resulting fabric will be to the touch In this respect, the use of fibers of greater than about 6.6 dtex [6 denier] is not preferred in absorbent article top sheets as such fibers undesirably decrease the softness of the resulting fabric to a level which may cause skin irritation during use.

Hydrophobic thermoplastic fibers useful in the first and second layers of the invention may be formed of hydrophobic thermoplastic material such as nylon 6, nylon 6.6, polyester, polyethylene, polypropylene and the like. However, polyethylene And polypropylene are generally preferred because of the lower melting points of these materials. Moreover, to be useful in the formation of the top sheet of the invention, these hydrophobic thermoplastic fibers are typically coated with a spin finish which contains lubricants, antistats and wetting agents. The wetting agents facilitate the initial strike-through of liquids through the top sheet. Thermobondable polypropylene fibers particularly useful as the hydrophobic thermoplastic fibers of the invention are commercially available from AMOCO Fabrics and Fibers Co. of Atlanta, GA and Hercules Inc. of Oxford, GA in 2.0 dtex [1.8 denier] and 2.4 dtex [2.2 denier] sizes, respectively.

In addition to single component hydrophobic thermoplastic fibers, bi-component fibers made from two hydrophobic thermoplastic materials of different melting points can also be used in the present invention. Sheath/Core, side-by-side, and other types of bi-component fibers can be used. However, the preferred bi-component fibers are selected from the group consisting of sheath/core fibers of the following resin combinations polyethylene/polypropylene, polyethylene/polyester, polypropylene/polyester, and copolyester/polyester. Specific examples of such fibers are 1.9 dtex [1.7 denier] and 3.3 dtex [3 denier] polyethylene/polyester sheath/core fibers available for BASF CORPORATION as Products 1051 and 1050, respectively; 2.2 and 3.3 dtex [2 and 3 denier] copolyester/polyester sheath/core fibers available from CELANESE FIBERS as Type 354; and 1.7 and 3.3 dtex [1.5 and 3 denier] polyethylene/polypropylene sheath/core fibers available from CHORI AMERICA as Dalwabo NBF Type H. Such bi-component fibers may be particularly useful to provide the requisite strength to the top sheet of this invention when the total basis weight of the top sheet is reduced and the total quantity of natural hydrophilic fiber is maximized.

Furthermore, the hydrophobic thermoplastic fiber component of each of these layers may be made up of fibers of a single composition, or a blend of fibers of different compositions, which may or may not be the same for each of the layers. However, it is generally preferred that the hydrophobic thermoplastic fiber components of both layers be formed of fibers of a single composition. In this regard, it is generally most preferred that the hydrophobic thermoplastic fibers in both layers be polypropylene fibers.

Similarly, the natural hydrophilic fiber component of the second layer may be formed of fibers of a single composition or of a blend of fibers of different compositions. Natural hydrophilic fibers useful in the present invention include silk, wool, natural cellulosic fibers such as cotton or wood pulp, and manufactured fibers composed of regenerated cellulose such as rayon and acetate. Preferably, the hydrophilic fibers of the second layer are of a single composition selected from cotton or rayon.

The fabric of the invention preferably has a basis weight within the range of from about 12 to about 36 grams per square meter (g/m$^2$) [about 10 to about 30 grams per square yard (g/yd$^2$)]. At basis weights below about 12 g/m$^2$ [10 g/yd$^2$], the fabric typically lacks sufficient strength, especially when wet, to be useful as a top sheet in an absorbent article. Furthermore, at such basis weights, containment of the finely divided particles of superabsorbent materials typically used in such absorbent articles becomes a problem, as the particles of absorbent material migrate through the top sheet and escape. At basis weights above about 36 g/m$^2$ [30 g/yd$^2$], the cost of the fabric generally makes its use in such absorbent articles economically infeasible.

In addition to the basis weight of the fabric, the overall proportion of natural hydrophilic fibers to hydrophobic thermoplastic fibers in the fabric is somewhat limited by the strength requirements of an absorbent article top sheet. In this respect, it is preferred that the fabric have an overall weight ratio of hydrophobic thermoplastic fibers to natural hydrophilic fibers in the range of from about 30:70 to about 70:30, and more preferred in the range of from about 40:60 to about 60:40. In this respect, it is most preferred that the overall weight ratio of hydrophobic thermoplastic fibers to natural hydrophilic fibers be within the range of from about 60:40 to about 50:50. At weight ratios less than about 30:70, the resulting fabric may not have sufficient strength to be useful as an absorbent article top sheet, whereas, at weight ratios above about 70:30, the resulting fabric may contain an insufficient quantity of natural hydrophilic fiber to provide the comfort promised by the inclusion of these fibers or to provide a significant reduction in the nonbiodegradable waste generated by such top sheets.

Thermal bonding of the nonwoven webs can be accomplished by any method known in the art which generates melt-bonded thermoplastic filaments. For example, suitable bonding methods include calendaring, through air bonding, infrared bonding and ultrasonic bonding techniques. Preferably, however, tho webs are bonded together at a plurality of discrete regions by calendaring the composite web between opposed rolls, wherein the surface of at least one of the rolls is heated to a temperature above the softening point of the hydrophobic thermoplastic fibers. Suitable calendaring arrangements for forming this plurality of discrete bonded regions include calendaring between a smooth surfaced roll and a roll having a raised pattern on the surface thereof, or between helically engraved rolls as disclosed in US-A-3,542,634, incorporated herein by reference.

The present invention is further illustrated by the following nonlimiting examples wherein all parts and percentages are by weight unless otherwise indicated.

Illustrative Examples

Example 1

A carded web having a basis weight of approximately 15.6 g/m$^2$ [13 g/yd$^2$] and composed of a substantially homogeneous blend of 75 weight percent, 1.7 dtex [1.5 denier] per filament (dpf) rayon staple fiber (commercially available from B.A.S.F. Co. under the trade designation BASF 8171), and 25 weight percent, 2.4 [2.2] dpf polypropylene staple fiber (commercially available from Hercules Co. under the trade designation T185) was laid on a moving belt. This layer was overlaid with a carded web having a basis weight of approximately 9.6 g/m$^2$ [8 g/yd$^2$] and consisting of 100 percent of the same 2.4 [2.2] dpf polypropylene staple fiber used in the other layer. The two-layered assembly, having an overall weight ratio of polypropylene to rayon of 56:44, was then bonded via calendaring between a smooth surfaced steel roll maintained at 147°C [296°F] and a steel roll having a raised pattern on its surface and maintained at 149°C [300°F]. The rolls were compressed together under a pressure of 2680 kilograms per linear meter (kg/m) [150 pounds per linear inch (pli)], and the web was passed between the rolls at a speed of 1 meter per second [200 feet per minute].

Example 2

A carded web having a basis weight of approximately 19.7 g/m$^2$ [16.5 g/yd$^2$] and composed of a substantially homogeneous blend of 75 weight percent of the 1.7 [1.5] dpf rayon staple fiber used in Example 1 and 25 weight percent of the 2.4 [2.2] dpf polypropylene staple fiber used in Example 1 was laid on moving belt. This layer was overlaid with a carded web having a basis weight of approximately 11.4 g/m$^2$ [9.5 g/yd$^2$] and consisting of 100 percent of the 2.4 [2.2] dpf polypropylene staple fiber used in Example 1. The two-layered assembly, having an overall weight ratio of polypropylene to rayon of 56:44, was then bonded via calendaring between a smooth surfaced steel roll maintained at 166°C [330°F] and a steel roll having a raised pattern on its surface and maintained a 150°C [302°F]. The rolls were compressed together under a pressure of 2680 kg/m [150 pli], and the web was passed between the rolls at a speed of 1 meter per second [200 feet per minute].

Example 3

A carded web having a basis weight of approximately 17.3 g/m$^2$ [14.5 g/yd$^2$] and composed of a substantially homogeneous blend of 75 weight percent, 1.7 [1.5] dpf rayon staple fiber (commercially available from B.A.S.F. Co. under the trade designation BASF 8174), and 25 weight percent of the 2.4 [2.2] dpf polypropylene staple fiber used in Example 1 was laid on a moving belt. This layer was overlaid with a carded web having a basis weight of approximately 16.1 g/m$^2$ [13.5 g/yd$^2$] and consisting of 100 percent of the 2.4 [2.2] dpf polypropylene staple fiber used in Example 1. The two-layered assembly, had an overall weight ratio of polypropylene to rayon of 61:39, and was bonded and described in Example 2.

Example 4

A two-lay red assembly, having an overall weight ratio of polypropylene fibers to rayon fibers of 61:39, prepared as described above in Example 3, was bonded via passing the web between a first pair of rolls consisting of smooth surfaced steel roll maintained at 166.1° C [331° F] and a steel roll having a raised pattern on its surface and maintained at 149° C [301° F], and then passing the web between a second pair of rolls consisting of a smooth surfaced steel roll maintained at 151° C [304° F] and a steel roll having a raised pattern on its surface and maintained at 165.6° C [330° F]. The rolls in both sets were compressed together under a pressure of 2680 kg/m [150 pli], and the web was passed between the rolls at a speed of 1 meter per second [200 feet per minute].

Control Example A

A carded web having a basis weight of approximately 15.6 [13 g/yd$^2$] and composed of a substantially homogeneous blend of 50 weight percent of the 1.7 [1.5] dpf rayon staple fiber used in Example 1 and 50 weight percent of the 2.4 [2.2] dpf polypropylene staple fiber used in Example 1 was laid on a moving belt. This layer was overlaid with an identical carded web to form a two-layered assembly, having an overall weight ratio of polypropylene fibers to rayon fibers of 50:50. The two-layered assembly was bonded via calendaring between a smooth surfaced steel roll maintained at 157° C [315° F] and a steel roll having a raised pattern on its surface and maintained at 141° C [286° F]. The rolls were compressed together under a pressure of 2680 kg/m [150 pli], and the web was passed between the rolls at a speed of .91 meters per second [180 feet per minute].

The strip tensile strength, caliper, strike-through, surface rewet and absorption capacity properties of the samples prepared in the foregoing Examples were tested according to the procedures outlined below.

Strip Tensile Strength

Strip tensile strength was evaluated by breaking a one inch by seven inch long sample generally following ASTM D1682-64, the One-Inch Cut Strip Test. The instrument cross-head speed was set at 5 inches per minute and the gauge length was set at 5 inches. The tensile strength in both the machine direction ("MD") and the cross direction ("CD") was evaluated. The strip tensile strength or breaking load, reported in grams per inch, is the average of at least eight measurements.

Caliper (Under Compression)

Caliper was determined by measuring the distance between the top and the bottom surface of the sheet while the sheet was held under a compression loading of 12 grams per square meter [10 g/yd$^2$]. The result, reported in millimeters, is the average of ten measurements.

Strike-Through

Strike-through was evaluated by a method similar to that described in US-A-4,391,869 and US-A-4,041,951. Strike-through was measured as the time for 5 ml of synthetic urine solution, placed in the cavity of the strike-through plate, to pass through the sample fabric into an absorbent pad. The result, reported in seconds, is generally the average of four tests.

Surface Rewet

Surface rewet was evaluated by a method similar to that described in US-A-4,041,951 and US-A-4,391,869. Surface rewet, reported in grams, was evaluated by adding synthetic urine through the sample fabric into the absorbent pad until the absorbent pad was nearly saturated. Thus, the sample fabric was wet at the beginning of the surface wetness test. For results denoted as Surface Rewet A, the loading factor was slightly less than 4 grams of synthetic urine per gram of absorbent sample A uniform pressure loading of 3.45 kPa [0.5 psi] was then applied and the procedure concluded as disclosed in the above patents. For results denoted as Surface Rewet B, the loading factor was increased to slightly over 4 grams of synthetic urine per gram of absorbent sample so that the absorbent pad was saturated with synthetic urine. A uniform pressure loading of 6.90 kPa [1.0 psi] was then applied and the procedure concluded as disclosed in the above patents. The result, reported in grams, is generally the average of four tests.

Absorption Capacity

Absorption capacity was evaluated by measuring the amount of water absorbed by a loosely rolled fabric sample in a specified period of time. Rectangular specimens measuring 7.62 cm [3 inches] wide and having a length in the machine direction sufficient to yield a sample weight of about 5 grams were cut from the fabric samples to be tested. Each specimen was weighed, rolled into a loose roll and placed in a cylindrical wire basket having a 5 cm diameter and a length of 8 cm, with the 7.62 cm edge of the specimen parallel to the side of the basket. The basket was then weighed to determine the combined weight of the specimen and basket. The basket was then dropped into a container of distilled water from a height of 2.54 cm [1 inch]. After the basket had been submerged for 10 seconds, the basket was removed from the container and allowed to drain for 10 seconds. The container was then weighed again to determine the amount of water absorbed. The absorption capacity is reported as a percentage of the dry weight of the specimen. The result reported is generally the average of five tests.

The results of these tests are shown in Table I. As can be seen from the test results, the samples of the present invention prepared in Examples 1-4 had shorter strike-through times and lower amounts of surface rewet than the homogeneously blended sample of Control Example A. Furthermore, the samples of Examples 1, 3 and 4 each had a lower absorption capacity than the sample of Control Example A. This is an advantageous characteristic for a top sheet material since it is desired that the liquid to be absorbed pass through the top sheet and be absorbed in the layer of absorbent or superabsorbent material below.

Table 1

Surface Rewet[1]

| Sample | Basis Weight g/m² [g/yd²] | Strip Tensile Properties MP kg/m [g/in] | CD kg/m [g/in] | Caliper (Under Compression of 12 g/m² [10 g/yd²]) mm [mils] | Strike-Through[1] sec Upside | Downside | A 3.45 kPA [.5 psi] compression g Upside | Downside | B 6.90 kPA [1.0 psi] compression g Upside | Downside | Absorption Capacity g |
|---|---|---|---|---|---|---|---|---|---|---|---|
| Example 1 | 25.1 [21] | 23.78 [604] | 6.77 [172] | .241 [9.5] | 1.7 | 1.9 | 0.6 | 0.6 | 2.2 | 2.2 | 994 |
| Example 2 | 31.1 [26] | 23.66 [601] | 10.63 [270] | .290 [11.4] | 1.5 | 1.8 | 1.8 | 0.7 | 3.4 | 2.7 | 1039 |
| Example 3 | 33.5 [28] | 35.20 [894] | 16.46 [418] | .310 [12.2] | 1.6 | 1.6 | 0.2 | 0.2 | 1.9 | 1.9 | 910 |
| Example 4 | 31.1 [26] | 24.09 [612] | 7.48 [190] | .226 [8.9] | 2.2 | 2.0 | 2.1 | 2.0 | 3.5 | 3.3 | 700 |
| Control Example A | 31.1 [26] | 48.43 [1230] | 12.09 [307] | .229 [9.0] | 2.7 | 2.7 | 2.9 | 3.0 | 4.0 | 4.1 | 1022 |

[1]For the samples of Examples 1-4 the upside is the side corresponding to the 100% polypropylene layer and the downside is the side corresponding to the rayon/polypropylene blend.

## Claims

1. A two-layer, nonwoven fabric comprising:

8

EP 0 521 016 B1

(a) a continuous first layer comprising at least about 75 weight percent hydrophobic thermoplastic fibers, and

(b) a second layer comprising a blend of from about 20 to 70 weight percent hydrophobic thermoplastic fibers and from about 30 to about 80 weight percent natural hydrophilic fibers, wherein said layers are secured together at a plurality of discrete regions by bonds formed of melt-fused portions of said hydrophobic thermoplastic fibers.

2. A two-layer, nonwoven fabric as recited in claim 1 wherein said hydrophobic thermoplastic fibers in said first and second layers are independently selected from the group consisting of nylon fibers, polyester fibers, polyethylene fibers, polypropylene fibers, polyethylene/polyester bi-component fibers, polypropylene/polyester bi-component fibers, polyethylene/polypropylene bi-component fibers, copolyester/polyester bi-component fibers and mixtures thereof.

3. A two-layer, nonwoven fabric as recited in claim 1 wherein said hydrophobic thermoplastic fibers in said first and second layers are polypropylene fibers.

4. A two-layer, nonwoven fabric as recited in any one of claims 1 to 3 wherein said natural hydrophilic fibers are selected from the group consisting of acetate fibers, rayon fibers, cotton fibers, wool fibers, silk fibers, wood pulp and mixtures thereof.

5. A two-layer, nonwoven fabric as recited in any one of claims 1 to 3 wherein said natural hydrophilic fibers are selected from the group consisting of cotton fibers and rayon fibers.

6. A two-layer, nonwoven fabric as recited in any one of claims 1 to 3 wherein said natural hydrophilic fibers are rayon fibers.

7. A two-layer, nonwoven fabric as recited in any one of claims 1 to 6 wherein said first layer comprises at least about 95 weight percent hydrophobic thermoplastic fibers.

8. A two-layer, nonwoven fabric as recited in any one of claims 1 to 6 wherein said continuous first layer consists essentially of polypropylene fibers.

9. A two-layer, nonwoven fabric as recited in any one of claims 1 to 8 wherein said second layer comprises a blend of from about 25 to 50 weight percent hydrophobic thermoplastic fibers and from about 50 to about 75 weight percent natural hydrophilic fibers.

10. A two-layer, nonwoven fabric as recited in claim 1 wherein said first layer comprises at least about 95 weight percent polypropylene fibers and said second layer comprises a blend of from about 25 to 50 weight percent polypropylene fibers and from about 50 to about 75 weight percent rayon fibers.

11. A two-layer, nonwoven fabric as recited in claim 10 wherein said polypropylene fibers and said rayon fibers are from 1.1 to 6.6 dtex (1 to 6 denier).

12. A two-layer, nonwoven fabric as recited in claim 10 wherein said polypropylene fibers and said rayon fibers are from 1.1 to 3.3 dtex (1 to 3 denier).

13. A two-layer, nonwoven fabric as recited in any one of claims 10 to 12 wherein said fabric has a basis weight within the range of from about 12 to about 36 $g/m^2$ (about 10 to about 30 $g/yd^2$).

14. A two-layer, nonwoven fabric as recited in any one of claims 10 to 13 wherein said fabric has an overall weight ratio of polypropylene fibers to rayon fibers within the range of from about 40:60 to about 60:40.

15. A two-layer, nonwoven fabric as recited in any one of claims 10 to 13 wherein said fabric has an overall weight ratio of polypropylene fibers to rayon fibers within the range of from about 50:50 to about 60:40.

16. A method of making a two-layer, nonwoven fabric comprising the steps of:

(a) forming a first, continuous, nonwoven, fibrous web comprising at least about 75 weight percent hydrophobic thermoplastic fibers;

9

(b) forming a second, nonwoven, fibrous web comprising a blend of from about 20 to 70 weight percent hydrophobic thermoplastic fibers and from about 30 to 80 weight percent natural hydrophilic fibers;

(c) forming a composite web by arranging said first and second webs in an overlying adjacent relationship;

(d) thermally bonding said first and second nonwoven webs together at a plurality of discrete regions by causing melt-fusing of said hydrophobic thermoplastic fibers; and

(e) allowing said composite web to cool to resolidify the melt-fused thermoplastic fibers and strengthen the bonds formed between said layers.

17. A method as recited in claim 16 wherein said first and second nonwoven webs are thermally bonded together by calendaring said composite web between a smooth surfaced roll and a roll having a raised pattern of the surface thereof, wherein the surface of at least one of said rolls is heated to a temperature above the softening point of said hydrophobic thermoplastic fiber.

18. A method as recited in claim 16 or 17 wherein said first nonwoven web consists essentially of said hydrophobic thermoplastic fibers and said second nonwoven web consists essentially of a blend of from about 25 to 50 weight percent hydrophobic thermoplastic fibers and from about 50 to about 75 weight percent natural hydrophilic fibers.

19. A method as recited in any one of claims 16 to 18 wherein said hydrophobic thermoplastic fibers in said first and second nonwoven webs are independently selected from the group consisting of nylon fibers, polyester fibers, polyethylene fibers, polypropylene fibers, polyethylene/polyester bi-component fibers, polypropylene/polyester bi-component fibers, polyethylene/polypropylene bi-component fibers, copolyester/polyester bi-component fibers and mixtures thereof; and said natural hydrophilic fibers are selected from the group consisting of acetate fibers, wool fibers, cotton fibers, rayon fibers, silk fibers, wood pulp and mixtures thereof.

20. A method as recited in any one of claims 16 to 18 wherein said hydrophobic thermoplastic fibers are polypropylene fibers and said natural hydrophilic fibers are rayon fibers.

21. A disposable diaper comprising a top sheet layer of the nonwoven fabric of any one of claims 1 to 15 attached to one side of a layer of absorbent material, and an impermeable outer covering attached to the side of said layer of absorbent material opposite said top sheet.

**Patentansprüche**

1. Zweilagiger, nicht gewebter Stoff mit:
   a) einer kontinuierlichen ersten Lage enthaltend mindestens etwa 75 Gew.-% hydrophobe thermoplastische Fasern, und
   b) einer zweiten Lage enthaltend eine Mischung aus etwa 20 bis 70 Gew.-% hydrophobe thermoplastische Fasern und etwa 30 bis etwa 80 Gew.-% natürliche, hydrophile Fasern, wobei die Lagen an einer Mehrzahl von einzelnen Bereichen aneinander durch Verbindungsstellen befestigt sind, die aus schmelzverschweißten Bereichen der hydrophoben thermoplastischen Fasern gebildet sind.

2. Zweilagiger, nicht gewebter Stoff gemäß Anspruch 1, wobei die hydrophoben, thermoplastischen Fasern in den ersten und zweiten Lagen unabhängig voneinander ausgewählt sind aus der Gruppe, die besteht aus Nylonfasern, Polyesterfasern, Polyäthylenfasern, Polypropylenfasern, Polyäthylen/Polyester-Zweikomponentenfasern, Polypropylen/Polyester-Zweikomponentenfasern, Polyäthylen/Polypropylen-Zweikomponentenfasern, Copolyester/Polyester-Zweikomponentenfasern und Mischungen daraus.

3. Zweilagiger, nicht gewebter Stoff gemäß Anspruch 1, wobei die hydrophoben, thermoplastischen Fasern in den ersten und zweiten Lagen Polypropylenfasern sind.

4. Zweilagiger, nicht gewebter Stoff gemäß einem der Ansprüche 1 bis 3, wobei die natürlichen, hydrophilen Fasern ausgewählt sind aus der Gruppe, die besteht aus Acetatfasern, Rayonfasern, Baumwollfasern, Wollfasern, Seidenfasern, Holzpulpe und Mischungen daraus.

5. Zweilagiger, nicht gewebter Stoff gemäß einem der Ansprüche 1 bis 3, wobei die natürlichen, hydrophilen Fasern ausgewählt sind aus der Gruppe, die aus Baumwollfasern und Ryonfasern besteht.

6. Zweilagiger, nicht gewebter Stoff gemäß einem der Ansprüche 1 bis 3, wobei die natürlichen, hydrophilen Fasern Rayonfasern sind.

7. Zweilagiger, nicht gewebter Stoff gemäß einem der Ansprüche 1 bis 6, wobei die erste Lage mindestens etwa 95 Gew.-% hydrophobe, thermoplastische Fasern enthält.

8. Zweilagiger, nicht gewebter Stoff gemäß einem der Ansprüche 1 bis 6, wobei die kontinuierliche, erste Lage im wesentlichen aus Polypropylenfasern besteht.

9. Zweilagiger, nicht gewebter Stoff gemäß einem der Ansprüche 1 bis 8, wobei die zweite Lage eine Mischung aus etwa 25 bis 50 Gew.-% hydrophoben, thermoplastischen Fasern und etwa 50 bis etwa 75 Gew.-% natürlicher, hydrophiler Fasern enthält.

10. Zweilagiger, nicht gewebter Stoff gemäß Anspruch 1, wobei die erste Lage mindestens etwa 95 Gew.-% Polypropylenfasern enthält und wobei die zweite Lage eine Mischung aus etwa 25 bis 50 Gew.-% Polypropylenfasern und etwa 50 bis etwa 75 Gew.-% Rayonfasern enthält.

11. Zweilagiger, nicht gewebter Stoff gemäß Anspruch 10, wobei die Polypropylenfasern und die Rayonfasern von 1,1 bis 6,6 dtex (1 bis 6 den) aufweisen.

12. Zweilagiger, nicht gewebter Stoff gemäß Anspruch 10, wobei die Polypropylenfasern und die Rayonfasern von 1,1 bis 3,3 dtex (1 bis 3 den) aufweisen.

13. Zweilagiger, nicht gewebter Stoff gemäß einem der Ansprüche 10 bis 12, wobei der Stoff ein Grundgewicht im Bereich von etwa 12 bis etwa 36 $g/m^2$ (etwa 10 bis 30 $g/yd^2$) aufweist.

14. Zweilagiger, nicht gewebter Stoff gemäß einem der Ansprüche 10 bis 13, wobei der Stoff ein Gesamt-Gewichtsverhältnis der Polypropylenfasern zu den Rayonfasern im Bereich von etwa 40:60 bis etwa 60:40 aufweist.

15. Zweilagiger, nicht gewebter Stoff gemäß einem der Ansprüche 10 bis 13, wobei der Stoff eine Gesamt-Gewichtsverhältnis der Polypropylenfasern zu den Rayonfasern im Bereich von etwa 50:50 bis etwa 60:40 aufweist.

16. Verfahren zum Herstellen eines zweilagigen, nicht gewebten Stoffes mit den folgenden Verfahrensschritten:
    a) Ausbilden einer ersten, kontinuierlichen, nicht gewebten Faserbahn mit mindestens etwa 75 Gew.-% hydrophoben, thermoplastischen Fasern;
    b) Ausbilden einer zweiten, nicht gewebten Faserbahn enthaltend eine Mischung aus etwa 20 bis 70 Gew.-% hydrophoben, thermoplastischen Fasern und etwa 30 bis 80 Gew.-% natürlichen, hydrophilen Fasern;
    c) Ausbilden einer zusammengesetzten Bahn durch Anordnen der ersten und der zweiten Bahnen in einer übereinanderliegenden, benachbarten Anordnung;
    d) Verbinden unter Wärmeeinwirkung der ersten und zweiten, nicht gewebten Bahnen miteinander an einer Mehrzahl von einzelnen Bereichen, indem ein Schmelzverschweißen der hydrophoben, thermoplastischen Fasern bewirkt wird; und
    e) Abkühlenlassen der zusammengesetzten Bahn, um die schmelzverschweißten thermoplastischen Fasern wieder zu erhärten und die zwischen den Lagen ausgebildeten Verbindungen zu festigen.

17. Verfahren gemäß Anspruch 16, wobei die ersten und zweiten nicht gewebten Bahnen unter Wärmeeinwirkung miteinander verbunden werden, indem die zusammengesetzte Bahn zwischen einer Rolle mit glatter Oberfläche und einer Rolle mit einem erhabenen Muster auf ihrer Oberfläche geprägt wird, wobei die Oberfläche von mindestens einer der Rollen auf eine Temperatur über dem Erweichungspunkt der hydrophoben thermoplastischen Fasern erwärmt wird.

**18.** Verfahren gemäß Anspruch 16 oder 17, wobei die erste nicht gewebte Bahn im wesentlichen aus den hydrophoben thermoplastischen Fasern und die zweite nicht gewebte Bahn im wesentlichen aus einer Mischung von etwa 25 bis 50 Gew.-% hydrophoben thermoplastischen Fasern und etwa 50 bis etwa 75 Gew.-% natürlichen, hydrophilen Fasern besteht.

**19.** Verfahren nach einem der Ansprüche 16 bis 18, wobei die hydrophoben thermoplastischen Fasern in der ersten und der zweiten nicht gewebten Bahn unabhängig voneinander aus der Gruppe ausgewählt werden, die besteht aus Nylonfasern, Polyesterfasern, Polyäthylenfasern, Polypropylenfasern, Polyäthylen/Polyester-Zweikomponentenfasern, Polypropylen/Polyester-Zweikomponentenfasern, Polyäthylen/Polypropylen-Zweikomponentenfasern, Copolyester/Polyester-Zweikomponentenfasern und Mischungen daraus, und wobei die natürlichen hydrophilen Fasern ausgewählt werden aus der Gruppe die besteht aus Acetatfasern, Wollfasern, Baumwollfasern, Rayonfasern, Seidenfasern, Holzpulpe und Mischungen daraus.

**20.** Verfahren nach einem der Ansprüche 16 bis 18, wobei die hydrophoben, thermoplastischen Fasern Polypropylenfasern und die natürlichen, hydrophilen Fasern Rayonfasern sind.

**21.** Wegwerfwindel mit einer oberen Flächenmateriallage aus dem nicht gewebten Stoff nach einem der Ansprüche 1 bis 15, die an einer Seite einer Lage aus absorbierendem Material angeordnet ist, und einer undurchlässigen, äußeren Bedeckung, die an der Seite der Lage aus absorbierendem Material angeordnet ist, die den oberen Flächenmaterial gegenüberliegt.

**Revendications**

**1.** Tissu non tissé à deux couches comprenant:
(a) une première couche continue comprenant au moins environ 75 pour cent en poids de fibres thermoplastiques hydrophobes, et
(b) une deuxième couche comprenant un mélange d'environ 20 à 70 pour cent en poids de fibres thermoplastiques hydrophobes et d'environ 30 à environ 80 pour cent en poids de fibres naturelles hydrophiles, dans lequel lesdites couches sont fixées l'une à l'autre en une pluralité de régions discrètes par des liens formés de parties fusibles desdites fibres thermoplastiques hydrophobes.

**2.** Tissu non tissé à deux couches selon la revendication 1, dans lequel lesdites fibres thermoplastiques hydrophobes desdites première et deuxième couches sont sélectionnées indépendamment dans un groupe constitué de fibres de nylon, de fibres de polyester, de fibres de polyéthylène, de fibres de polypropylène, de fibres à deux constituants de polyéthylène/polyester, de fibres à deux constituants de polypropylène/polyester, de fibres à deux constituants de polyéthylène/polypropylène, de fibres à deux constituants de copolyester/polyester et de leurs mélanges.

**3.** Tissu non tissé à deux couches selon la revendication 1, dans lequel lesdites fibres thermoplastiques hydrophobes desdites première et deuxième couches sont des fibres de polypropylène.

**4.** Tissu non tissé à deux couches selon l'une quelconque des revendications 1 à 3, dans lequel lesdites fibres naturelles hydrophiles sont sélectionnées dans le groupe constitué de fibres d'acétate, de fibres de rayonne, de fibres de coton, de fibres de laine, de fibres de soie, de pâte à papier, et de leurs mélanges.

**5.** Tissu non tissé à deux couches selon l'une quelconque des revendications 1 à 3, dans lequel lesdites fibres naturelles hydrophiles sont sélectionnées dans le groupe constitué de fibres de coton et de fibres de rayonne.

**6.** Tissu non tissé à deux couches selon l'une quelconque des revendications 1 à 3, dans lequel lesdites fibres naturelles hydrophiles sont des fibres de rayonne.

**7.** Tissu non tissé à deux couches selon l'une quelconque des revendications 1 à 6, dans lequel ladite première couche comprend au moins 95 pour cent en poids de fibres thermoplastiques hydrophobes.

**8.** Tissu non tissé à deux couches selon l'une quelconque des revendications 1 à 6, dans lequel ladite première couche continue est essentiellement constituée de fibres de polypropylène.

**9.** Tissu non tissé à deux couches selon l'une quelconque des revendications 1 à 8, dans lequel ladite deuxième couche comprend un mélange d'environ 25 à 50 pour cent en poids de fibres thermoplastiques hydrophobes et d'environ 50 à 75 pour cent en poids de fibres naturelles hydrophiles.

**10.** Tissu non tissé à deux couches selon la revendication 1, dans lequel ladite première couche comprend au moins environ 95 pour cent en poids de fibres de polypropylène et ladite deuxième couche comprend un mélange d'environ 25 à 50 pour cent en poids de fibres de polypropylène et d'environ 50 à environ 75 pour cent en poids de fibres de rayonne.

**11.** Tissu non tissé à deux couches selon la revendication 10, dans lequel lesdites fibres de polypropylène et lesdites fibres de rayonne ont un dtex de 1,1 à 6,6 (1 à 6 deniers).

**12.** Tissu non tissé à deux couches selon la revendication 10, dans lequel lesdites fibres de polypropylène et lesdites fibres de rayonne ont un dtex de 1,1 à 3,3 (1 à 3 deniers).

**13.** Tissu non tissé à deux couches selon l'une quelconque des revendications 10 à 12, dans lequel ledit tissu a un poids de base dans l'intervalle d'environ 12 à 36 g/m$^2$ (environ 10 à environ 30 g/yd$^2$).

**14.** Tissu non tissé à deux couches selon l'une quelconque des revendications 10 à 13, dans lequel ledit tissu a un rapport pondéral global des fibres de polypropylène aux fibres de rayonne se situant dans l'intervalle d'environ 40:60 à environ 60:40.

**15.** Tissu non tissé à deux couches selon l'une quelconque des revendications 10 à 13, dans lequel ledit tissu a un rapport pondéral global des fibres de polypropylène aux fibres de rayonne se situant dans l'intervalle d'environ 50:50 à environ 60:40.

**16.** Procédé de fabrication d'un tissu non tissé à deux couches comprenant les étapes consistant à:
(a) préparer un premier voile fibreux continu non tissé comprenant au moins 75 pour cent en poids de fibres thermoplastiques hydrophobes;
(b) préparer un second voile fibreux non tissé comprenant un mélange d'environ 20 à 70 pour cent en poids de fibres thermoplastiques hydrophobes et d'environ 30 à 80 pour cent en poids de fibres naturelles hydrophiles;
(c) préparer un voile composite en disposant lesdits premier et deuxième voiles de façon adjacente et superposés l'un à l'autre;
(d) lier thermiquement lesdits premier et second voiles non tissés l'un à l'autre en une pluralité de régions discrètes en faisant fondre lesdites fibres thermoplastiques hydrophobes; et
(e) permettre audit voile composite de se refroidir afin de solidifier de nouveau les fibres thermoplastiques fusibles et de renforcer les liens formés entre lesdites couches.

**17.** Procédé selon la revendication 16, dans lequel lesdits premier et deuxième voiles non tissés sont thermiquement liés l'un à l'autre par calandrage dudit voile composite entre un rouleau à surface lisse et un rouleau ayant un motif en relief sur sa surface, la surface d'au moins l'un desdits rouleaux étant chauffée à une température supérieure au point de ramollissement de ladite fibre thermoplastique hydrophobe.

**18.** Procédé selon la revendication 16 ou 17, dans lequel ledit premier voile non tissé est essentiellement constitué desdites fibres thermoplastiques hydrophobes et ledit deuxième voile non tissé est essentiellement constitué d'un mélange d'environ 25 à 50 pour cent en poids de fibres thermoplastiques hydrophobes, et d'environ 50 à environ 75 pour cent en poids de fibres naturelles hydrophiles.

**19.** Procédé selon l'une quelconque des revendications 16 à 18, dans lequel lesdites fibres thermoplastiques hydrophobes desdits premier et deuxième voiles non tissés sont indépendamment sélectionnées dans le groupe constitué de fibres de nylon, de fibres de polyester, de fibres de polyéthylène, de fibres de polypropylène, de fibres à deux constituants de polyéthylène/polyester, de fibres à deux constituants de polypropylène/polyester, de fibres à deux constituants de polyéthylène/polypropylène, de fibres à deux constituants de copolyester/polyester et de leurs mélanges, et lesdites fibres naturelles hydrophiles sont sélectionnées dans le groupe constitué de fibres d'acétate, de fibres de laine, de fibres de coton, de fibres de rayonne, de fibres de soie, de pâte à papier et de leurs mélanges.

20. Procédé selon l'une quelconque des revendications 16 à 18, dans lequel lesdites fibres thermoplastiques hydrophobes sont des fibres de polypropylène, et lesdites fibres naturelles hydrophiles sont des fibres de rayonne.

21. Couche jetable comprenant une couche formant feuille supérieure en tissu non tissé selon l'une quelconque des revendications 1 à 15, fixée à une face d'une couche de matériau absorbant et un revêtement extérieur imperméable fixé à la face de ladite couche de matériau absorbant qui est opposée à ladite feuille supérieure.